# EUROPEAN PATENT APPLICATION

(11) **EP 4 850 541 A1**
(43) Date of publication of application: **09.12.2026**
(21) Application number: 25772938.4
(22) Date of filing: 11.03.2025
(51) Int. Cl.: A24F 40/10, A24F 40/40, A24F 40/42

(54) **ELECTRONIC ATOMIZATION APPARATUS AND LIQUID STORAGE DEVICE FOR ELECTRONIC ATOMIZATION APPARATUS**

(30) Priority: 22.03.2024 CN 202410350542
(71) Applicant: Shenzhen First Union Technology Co., Ltd., Shenzhen, Guangdong 518000 (CN)
(72) Inventor: HUANG, Jiayin, Shenzhen, Guangdong 518000 (CN); YUE, Zhaojin, Shenzhen, Guangdong 518000 (CN); ZHOU, Xiangxiong, Shenzhen, Guangdong 518000 (CN); XU, Zhongli, Shenzhen, Guangdong 518000 (CN); LI, Yonghai, Shenzhen, Guangdong 518000 (CN)
(74) Representative: Ran, Handong
(86) International application number: PCT/CN2025/081863
(87) International publication number: WO 2025/195230

(57) **Abstract**

The present application sets forth an electronic atomization apparatus, and a liquid storage device for the electronic atomization apparatus. The electronic atomization apparatus comprises: a first main body and a second main body; the first main body comprises a first liquid storage chamber and at least one liquid output connector; the second main body comprises a second liquid storage chamber, an atomization assembly, and at least one liquid input port; when the first main body is joined to the second main body, the liquid output connector is at least partially inserted into the liquid input port, and establishes a capillary channel between the two; the capillary channel is capable of adsorbing and retaining a liquid matrix output from the liquid output connector by means of capillary action, and replenishing the adsorbed and retained liquid matrix into the second liquid storage chamber when a change in a gas pressure difference between the second liquid storage chamber and the first liquid storage chamber occurs. In the described electronic atomization apparatus, the first liquid storage chamber in the first main body is capable of replenishing a predetermined amount of liquid matrix into the second liquid storage chamber in the second main body; the above liquid matrix replenishment operation is performed automatically, without requiring manual intervention.

## Description

### CROSS-REFERENCE TO RELATED APPLICATIONS

The present application claims priority to Chinese Application No. 202410350542.5, entitled "ELECTRONIC ATOMIZATION DEVICE AND RESERVOIR FOR ELECTRONIC ATOMIZATION DEVICE" filed with the China National Intellectual Property Administration on March 22, 2024, which is incorporated by reference in its entirety.

### TECHNICAL FIELD

Embodiments of this application relate to the field of electronic atomization technologies, and in particular, to an electronic atomization device and a reservoir for an electronic atomization device.

### BACKGROUND

Tobacco products (such as cigarettes and cigars) burn tobacco during use to produce tobacco smoke. People try to replace these tobacco-burning products by manufacturing products that release compounds without burning.

An example of such products is a heating device that releases a compound by heating rather than burning a material. For example, the material may be tobacco or another non-tobacco product, and the non-tobacco product may or may not include nicotine. In another example, there are aerosol-providing articles, such as electronic atomization devices. These devices usually include a liquid, and the liquid is heated to vaporize, to generate an inhalable aerosol. The liquid may include nicotine and/or fragrance and/or aerosol-generating substances (for example, glycerol). In a known electronic atomization device, a reusable body is supplemented with a liquid substrate through an independently replaceable liquid source.

### SUMMARY

An embodiment of this application provides an electronic atomization device, including:
a first body and a second body capable of existing independently and allowing a user operation to combine the first body with the second body, where
the first body includes:
   a first liquid storage cavity, for storing a liquid substrate; and
   at least one liquid output connector, configured to output the liquid substrate stored in the first liquid storage cavity; and
   the second body includes:
      a second liquid storage cavity, for storing a liquid substrate;
      an atomization assembly, for receiving and atomizing the liquid substrate from the second liquid storage cavity to generate an aerosol; and
      at least one liquid input interface, communicating with the second liquid storage cavity, where
      when the first body is combined with the second body, the liquid output connector is at least partially inserted into the liquid input interface and a capillary channel is established between the liquid output connector and the liquid input interface; and the capillary channel is configured to adsorb and retain, by means of a capillarity action, the liquid substrate output from the liquid output connector, and supplement the second liquid storage cavity with the adsorbed and retained liquid substrate when an air pressure difference between the second liquid storage cavity and the first liquid storage cavity changes.

In some embodiments, when the first body is combined with the second body, the inner surface of the liquid input interface at least partially surrounds an outer side surface of the liquid output connector, and the capillary channel is defined by a first distance between the inner surface of the liquid input interface and the outer side surface of the liquid output connector.

In some embodiments, when the first body is combined with the second body, a first distance between the inner surface of the liquid input interface and an outer side surface of the liquid output connector is in a range from 0.1 mm to 1.5 mm.

In some embodiments, the liquid output connector has a closed free end and a side surface connected to the free end and a liquid outlet for outputting a liquid substrate is provided on the side surface.

In some embodiments, a diameter or a width of the liquid outlet is greater than the first distance.

In some embodiments, a diameter or a width of the liquid outlet is in a range from 0.5 mm to1.5 mm.

In some embodiments, the liquid output connector is hollow and tubular, and a liquid output channel in communication with the first liquid storage cavity is defined in the liquid output connector; and
an inner diameter of the liquid output connector is greater than a diameter or a width of the liquid outlet.

In some embodiments, an inner diameter of the liquid output connector is in a range of 1.5-3.0 mm.

In some embodiments, at least two liquid outlets are provided on the liquid output connector; and
when one of the at least two liquid outlets outputs the liquid substrate of the first liquid storage cavity, the other liquid outlet is configured as an air inlet for air to enter the first liquid storage cavity.

In some embodiments, at least two liquid outlets are provided on the liquid output connector; and
the at least two liquid outlets are arranged opposite to each other along a radial direction of the liquid output connector.

In some embodiments, the second body further includes:
a liquid retaining element, arranged in the second liquid storage cavity, for adsorbing and retaining the liquid substrate in the second liquid storage cavity; and
the atomization assembly is arranged to draw or receive the liquid substrate from the liquid retaining element.

In some embodiments, the liquid retaining element abuts against a port of the liquid input interface that is located in the second liquid storage cavity.

In some embodiments, when the first body is combined with the second body, a second distance is maintained between the liquid output connector and the liquid retaining element, so that the liquid output connector and the liquid retaining element are not in contact.

In some embodiments, the second distance is in a range from 0.1 mm to 1.5 mm.

In some embodiments, the second body further includes:
a first end and a second end facing away from each other along a longitudinal direction, where the first body is capable of being combined with the second body at the first end;
the second liquid storage cavity has a first inner wall close to the first end; and
a first compartment is provided between the liquid retaining element and the first inner wall.

In some embodiments, the second liquid storage cavity further has a second inner wall close to the second end; and a second compartment is provided between the liquid retaining element and the second inner wall; and
the second compartment and the first compartment are in fluid communication.

In some embodiments, the second liquid storage cavity has an inner side wall that surrounds the second liquid storage cavity along a circumferential direction;
a convex ridge extending longitudinally is provided on the inner side wall; and
an outer side surface of the liquid retaining element abuts against the convex ridge, so that an air gap is formed between the outer side surface of the liquid retaining element and the inner side wall of the second liquid storage cavity.

In some embodiments, the second body further includes:
an airflow channel, for air to pass through the second body during inhalation; and
an air exchange channel, allowing the second liquid storage cavity and the airflow channel to be in air communication, for regulating pressure in the second liquid storage cavity.

In some embodiments, the second body further includes:
a support element, at least partially defining the second liquid storage cavity and the liquid input interface; and
a flexible second sealing element combined with the support element, where when the first body is combined with the second body, the second sealing element is at least partially located between the liquid output connector and the support element, for providing sealing between the liquid output connector and the support element.

In some embodiments, the air exchange channel is at least partially defined between the support element and the second sealing element.

In some embodiments, the air exchange channel includes a vent hole or a vent slot that is formed in the support element and/or the second sealing element.

In some embodiments, the second body further includes:
a first end and a second end facing away from each other along a longitudinal direction; and
a receiving cavity that is open at the first end, where when the first body is combined with the second body, the first body is at least partially received in the receiving cavity; and
the air exchange channel is formed or defined between the receiving cavity and the second liquid storage cavity.

In some embodiments, the second body further includes:
a tubular element, running through the second liquid storage cavity along a longitudinal direction, where
the atomization assembly is accommodated in the tubular element and includes:
   a liquid guide element, located in the tubular element and arranged to receive the liquid substrate from the second liquid storage cavity; and
   a heating element, combined with the liquid guide element and configured to heat at least part of the liquid substrate retained in the liquid guide element to generate an aerosol, where
   the liquid guide element and/or the heating element and/or the tubular element at least partially surrounds or defines the airflow channel.

In some implementations, an air outlet is provided in the first body;
an air inlet is provided in the second body; and
the airflow channel defines an airflow path running from the air inlet to the air outlet through the atomization assembly, to transfer the aerosol to the air outlet; and the airflow channel has one part defined by the first body and the other part defined by the second body.

In some embodiments, the second body further includes:
a first end and a second end facing away from each other along a longitudinal direction;
a receiving cavity that is open at the first end, where when the first body is combined with the second body, the first body is at least partially received in the receiving cavity; and
a cell, configured to supply power to the atomization assembly, where
the second liquid storage cavity is disposed between the receiving cavity and the cell.

In some embodiments, the liquid input interface is exposed to the receiving cavity.

In some embodiments, the second body further includes:
a control circuit board, arranged between the cell and the second end, and configured to control the cell to supply power to the atomization assembly.

In some embodiments, the second body further includes:
an electrical connection element, arranged between the second liquid storage cavity and the cell, where both the atomization assembly and the control circuit board are electrically connected to the electrical connection element, so that the electrical connection element establishes an electrical connection between the atomization assembly and the control circuit board.

In some embodiments, a volume of the first liquid storage cavity is greater than a volume of the second liquid storage cavity; and/or
the first liquid storage cavity is capable of storing a liquid substrate in a range from 5mL to 20 mL; and/or
the second liquid storage cavity is capable of storing a liquid substrate in a range from 0.5mL to 3 mL.

Another embodiment of this application further provides an electronic atomization device, including:
a first body and a second body capable of existing independently and allowing a user operation to combine the first body with the second body, where
the first body includes:
   a first liquid storage cavity, for storing a liquid substrate; and
   at least one liquid output connector, configured to output the liquid substrate stored in the first liquid storage cavity; and
   the second body includes:
      a second liquid storage cavity, for storing a liquid substrate;
      an atomization assembly, for receiving and atomizing the liquid substrate from the second liquid storage cavity to generate an aerosol; and
      at least one liquid input interface, communicating with the second liquid storage cavity, where
      when the first body is combined with the second body, the liquid output connector is at least partially inserted into the liquid input interface to allow the first liquid storage cavity and the second liquid storage cavity to be in communication, enabling the second liquid storage cavity to be supplemented with the liquid substrate from the first liquid storage cavity; and an inner surface of the liquid input interface at least partially surrounds an outer side surface of the liquid output connector, and a first distance from 0.1 mm to 1.5 mm is maintained between the inner surface of the liquid input interface and the outer side surface of the liquid output connector.

Another embodiment of this application further provides a reservoir for an electronic atomization device, including:
a first body, including a proximal end and a distal end facing away from each other along a longitudinal direction, where
a first liquid storage cavity for storing a liquid substrate is defined in the first body; and the first liquid storage cavity has an opening toward the distal end; and
a closing element connected to the first body, defines at least part of a boundary of the first liquid storage cavity, and closes the opening of the first liquid storage cavity, where
the closing element is provided with at least one liquid output connector extending toward the distal end; a liquid output channel is defined in the liquid output connector, and a liquid outlet is arranged on an outer side surface of the liquid output connector; and the liquid outlet is in fluid communication with the first liquid storage cavity through the liquid output channel, for outputting the liquid substrate from the first liquid storage cavity; and
a width or a diameter of the liquid outlet is less than an inner diameter of the liquid output channel.

Another embodiment of this application further provides a second body for an electronic atomization device, including:
a first end and a second end facing away from each other along a longitudinal direction;
a receiving cavity, open at the first end, for receiving a first body of the electronic atomization device;
a second liquid storage cavity, for storing a liquid substrate;
an atomization assembly, configured to receive the liquid substrate from the second liquid storage cavity and atomize the liquid substrate to generate an aerosol;
a cell, located between the second liquid storage cavity and the second end, and configured to supply power to the atomization assembly; and
at least one liquid input interface, exposed to the receiving cavity and communicating with the second liquid storage cavity, where when the first body of the electronic atomization device is received in the receiving cavity, the second liquid storage cavity can be supplemented with a liquid substrate through the liquid input interface.

In some embodiments, liquid injection holes are further provided in the support element, for injecting a liquid substrate into the second liquid storage cavity through the liquid injection holes; and the liquid injection hole and the liquid input interface are isolated from each other; and
a plug is arranged on the second sealing element; when the second sealing element is combined with the support element, the plug extends into the liquid injection hole to close or plug the liquid injection hole; and the second sealing element is also removable from the support element, to open the liquid injection hole.

According to the foregoing electronic atomization device, during use, the second liquid storage cavity in the second body can be supplemented with a predetermined amount of the liquid substrate in the first liquid storage cavity in the first body during an inhalation process of a user or a lag period of inhalation. The foregoing action of adding the liquid substrate is performed by the device automatically without human intervention.

### BRIEF DESCRIPTION OF THE DRAWINGS

One or more embodiments are exemplarily described with reference to the corresponding figures in the accompanying drawings, and the descriptions are not to be construed as limiting the embodiments. Elements in the accompanying drawings that have same reference numerals are represented as similar elements, and unless otherwise particularly stated, the figures in the accompanying drawings are not drawn to scale.
FIG. 1 is a schematic diagram of an electronic atomization device according to an embodiment.
FIG. 2 is a schematic structural diagram of the electronic atomization device in FIG. 1 from another perspective.
FIG. 3 is a schematic diagram of a first body and a second body in FIG. 1 in a separated state from one perspective.
FIG. 4 is a schematic diagram of the first body and the second body in FIG. 3 in the separated state from another perspective.
FIG. 5 is a schematic exploded view of a first body in FIG. 1 from one perspective.
FIG. 6 is a schematic exploded view of a first body in FIG. 1 from another perspective.
FIG. 7 is a schematic cross-sectional view of a first body in FIG. 1 from one perspective.
FIG. 8 is a schematic cross-sectional view of a second body in FIG. 1 from one perspective.
FIG. 9 is a schematic cross-sectional view of the second body in FIG. 8 from another perspective.
FIG. 10 is a schematic diagram of an atomization module in FIG. 9 before it is assembled in a second housing.
FIG. 11 is a schematic exploded view of a second body in FIG. 1 from one perspective.
FIG. 12 is a schematic exploded view of a second body in FIG. 1 from another perspective.
FIG. 13 is a schematic exploded view of a second body in FIG. 1 from one cross-sectional perspective.
FIG. 14 is a schematic cross-sectional view of the electronic atomization device in FIG. 1 from one perspective.
FIG. 15 is an enlarged view of a part B1 in FIG. 14.
FIG. 16 is a schematic structural diagram of a second sealing element in FIG. 13 from another perspective.
FIG. 17 is a schematic structural diagram of a support element in FIG. 13 from another perspective.
FIG. 18 is a schematic cross-sectional view of the support element in FIG. 17 from another perspective.
FIG. 19 is a schematic diagram of an injection operation performed after removal of a second sealing element in FIG. 4.
FIG. 20 is a schematic cross-sectional view of the electronic atomization device in FIG. 1 from another perspective.
FIG. 21 is an enlarged view of a part B2 in FIG. 20.
FIG. 22 is a schematic structural diagram of a heating element in FIG. 13 from another perspective.
FIG. 23 is a schematic exploded view of a support, a control circuit board, and a wrapping element in FIG. 13 before assembly from another perspective.

### DETAILED DESCRIPTION

To facilitate the understanding of this application, this application is described in more detail below with reference to accompanying drawings and specific implementations.

This application provides an electronic atomization device, which is configured to atomize a liquid substrate to generate an aerosol.

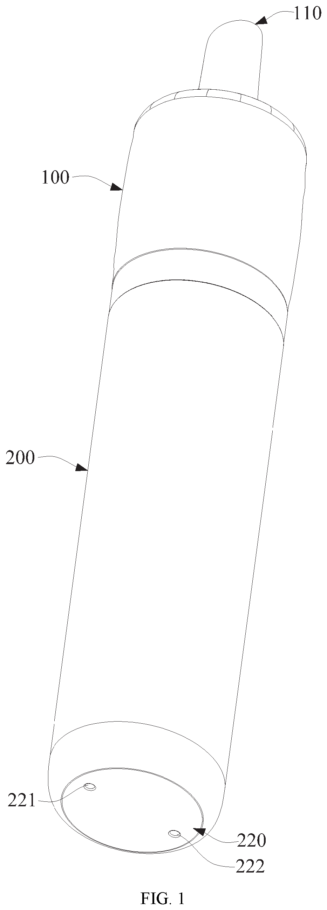

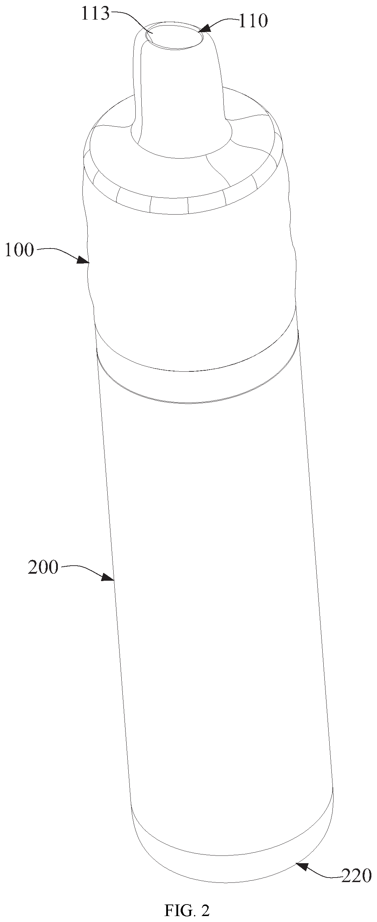

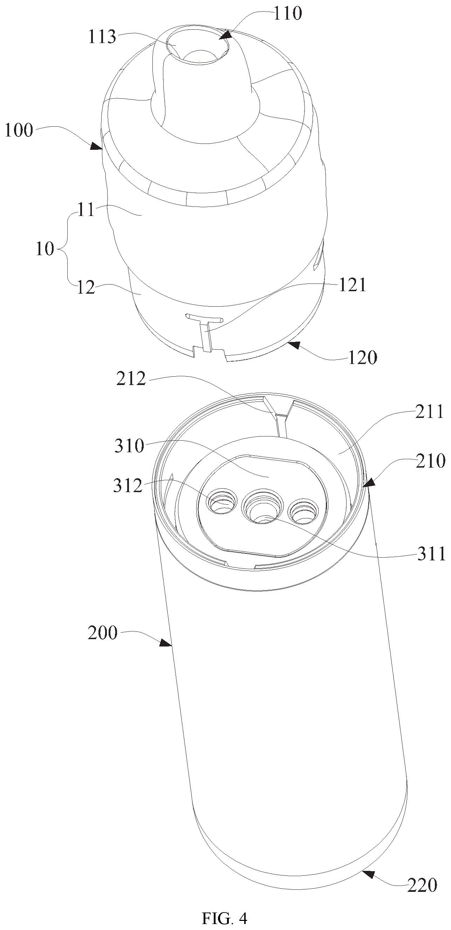

FIG. 1 to FIG. 4 are each a schematic diagram of an electronic atomization device according to an embodiment. In this embodiment, the electronic atomization device includes a first body 100 and a second body 200. The first body 100 and the second body 200 may both exist independently and can be combined with each other.

In an embodiment, the first body 100 can store a larger amount of liquid substrate than the second body 200, for supplementing the second body 200 with the liquid substrate during use. The second body 200 can store a relatively small amount of liquid substrate, and atomize the liquid substrate to generate an aerosol. Before being combined, the first body 100 and the second body 200 exist independently from each other. Moreover, after being combined, the first body 100 and the second body 200 jointly define a complete electronic atomization device, for use or inhalation of the aerosol by a user.

In some embodiments, when existing separately or independently, the first body 100 and the second body 200 cannot be independently provided for use or inhalation by a user. For example, as shown in FIG. 1 to FIG. 4, the first body 100 at least partially defines a suction nozzle for use or inhalation by a user. The second body 200 is capable of atomizing a liquid substrate to generate an aerosol. When the first body 100 is removed or separated from the second body 200, the first body 100 cannot separately atomize a liquid substrate to generate an aerosol, and the second body 200 cannot separately be provided for a user for inhalation. In some embodiments, the first body 100 and the second body 200 can be used by a user only when they are combined with each other to define a complete electronic atomization device, and are recycled as a whole after a liquid substrate inside them has been consumed.

Alternatively, in some other embodiments, the second body 200 is configured to atomize a liquid substrate to generate an aerosol. The first body 100 is removably combined with the second body 200. The first body 100 is used as a consumable material and thus is replaceable while the second body 200 is reusable. After the liquid substrate in the first body 100 has been consumed, the user may remove it from the second body 200 and replace it with a new first body 100.

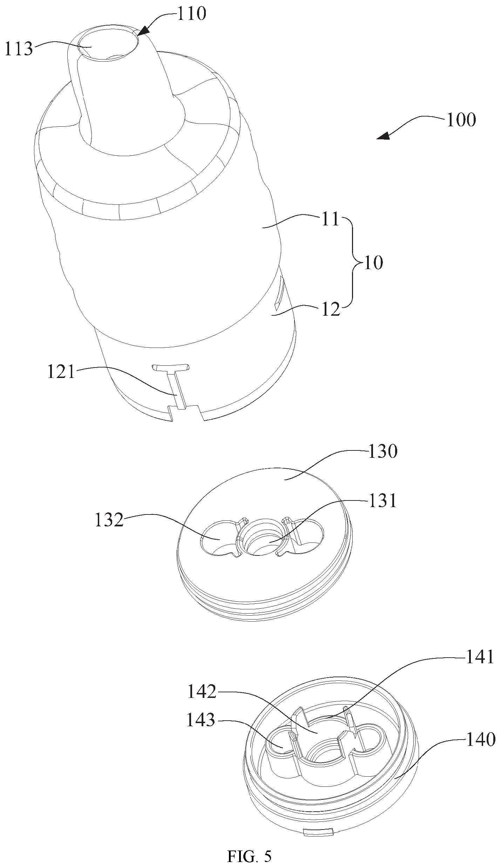

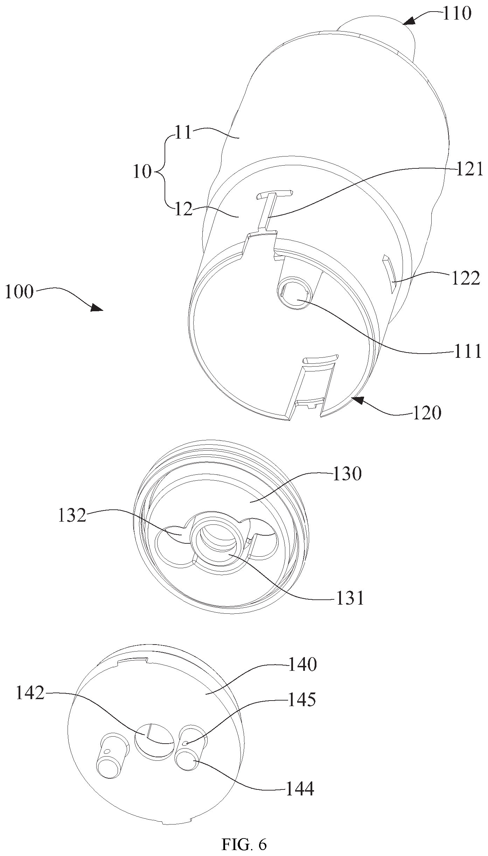

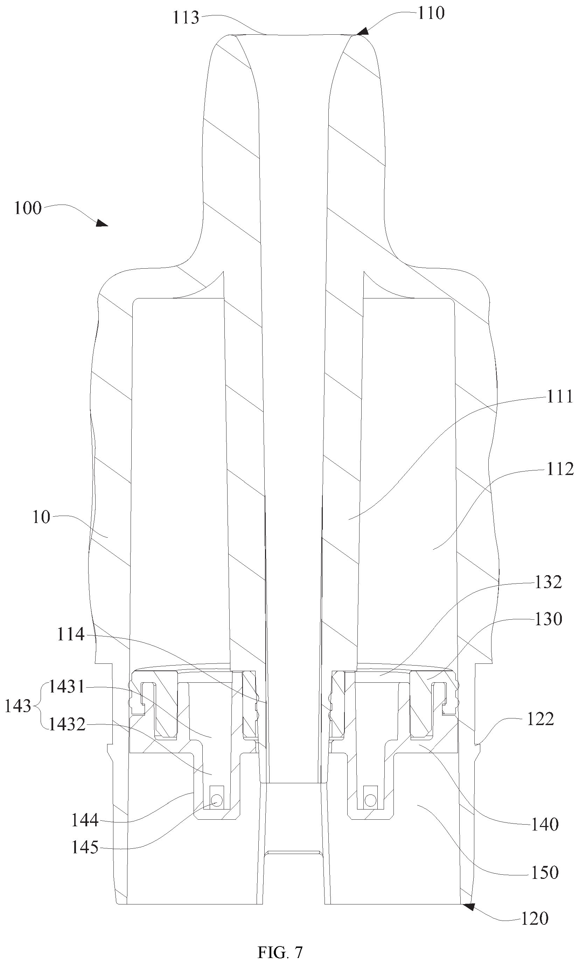

As shown in FIG. 1 to FIG. 7, the first body 100 includes a plurality of components disposed inside a first housing 10 (which may be referred to as a housing). An overall design of the first housing 10 may vary, and a type or configuration of the first housing 10 that may define an overall size and shape of the first body 100 may vary. Usually, the first housing 10 may be formed by a single integral housing, or the first housing 10 may be formed by two or more separable bodies. As shown in FIG. 1 to FIG. 7, the first housing 10 may include one or more reusable components. The first housing 10 has a proximal end 110 and a distal end 120 that are opposite to each other along a longitudinal direction. During use, the proximal end 110 is an end close to a position of inhalation by a user; and the distal end 120 is an end away from the user. In some examples, the first housing 10 may be entirely or only partially made of a metal or alloy such as stainless steel or aluminum, or other appropriate materials including various plastics (for example, polycarbonates), metal plating over plastics, ceramics, and the like.

As shown in FIG. 1 to FIG. 7, the distal end 120 of the first body 100 defined by the first housing 10 is open. Alternatively, in some other embodiments, the distal end 120 of the first body 100 may be provided with a detachable end cap, or a sealing film that can be torn off or removed, or the like, which closes the distal end 120 of the first body 100 before manufacturing, packaging, or use.

As shown in FIG. 1 to FIG. 7, the first housing 10 includes:
a first part 11 and a second part 12 sequentially arranged along a longitudinal direction. When the first body 100 is combined with the second body 200, the second part 12 is inserted or extended into the second body 200 while the first part 11 is located outside the second body 200. In this way, during use, a liquid substrate in a first liquid storage cavity 112 in the first part 11 is visible through the exposed first part 11 of the first housing 10, which is beneficial for the user to observe consumption or remaining of the liquid substrate in the first liquid storage cavity 112.

As shown in FIG. 1 to FIG. 7, at least one or more first guide structures 121 are arranged on an outer surface of the second part 12. Correspondingly, at least one or more second guide structures 211 are arranged on the second body 200. When the first body 100 is combined with the second body 200, the first guide structure 121 and the second guide structure 211 cooperate to provide guidance. As shown in FIG. 1 to FIG. 7, the first guide structure 121 includes a convex ridge located on the outer surface of the second part 12. The convex ridge of the first guide structure 121 is substantially arranged extending longitudinally. The second guide structure 211 is substantially a guide groove 211 extending longitudinally.

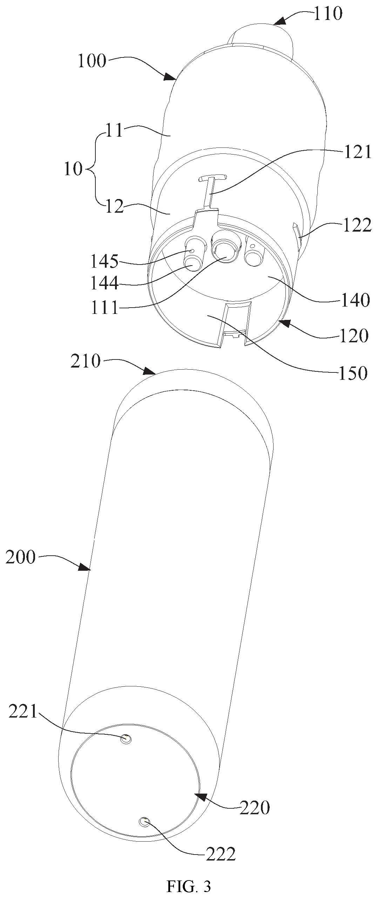

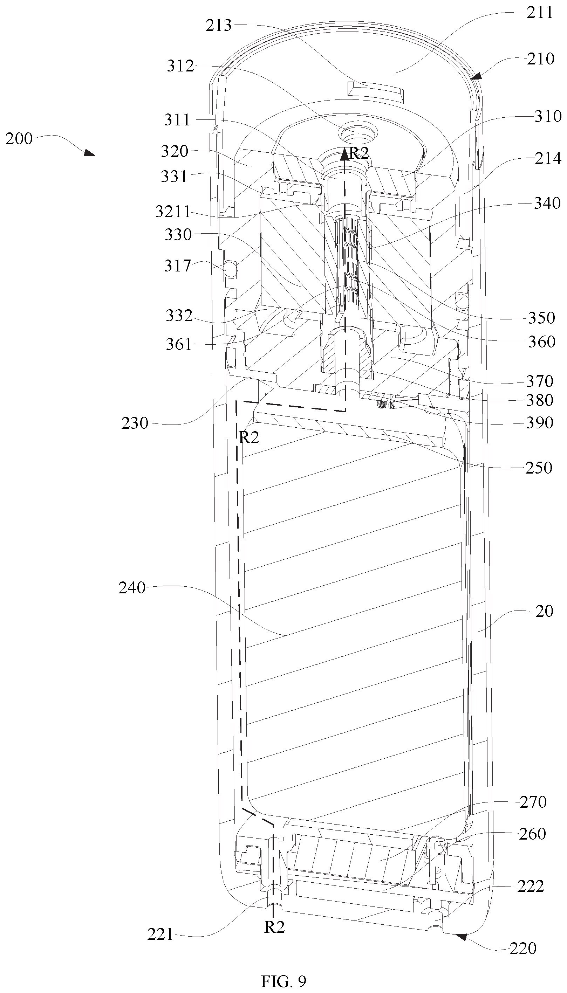

As shown in FIG. 1 to FIG. 9, at least one or more first connection structures 122 are further arranged on the outer surface of the second part 12. Correspondingly, a plurality of second connection structures 213 are arranged on the second body 200. When the first body 100 is combined with the second body 200, the second part 12 is inserted or extended into the second body 200, and the at least one or more first connection structures 122 and the second connection structures 213 cooperate to connect the first body 100 to the second body 200. In the embodiments shown in FIG. 3 to FIG. 9, the first connection structure 122 is, for example, a clamping protrusion; and the second connection structure 213 is, for example, a clamping groove.

As shown in FIG. 1 to FIG. 7, the first body 100 further includes:
an air outlet 113, located at the proximal end 110, for inhalation by the user;
an aerosol output tube 111, arranged extending from the air outlet 113 to the distal end 120, for delivering the aerosol to the air outlet 113, where in an embodiment, the aerosol output tube 111 is integrally formed with the first housing 10; and
the first liquid storage cavity 112, for storing the liquid substrate, where the first liquid storage cavity 112 is at least partially defined and formed between the aerosol output tube 111 and the first housing 10. A side of the first liquid storage cavity 112 close to the proximal end 110 is closed, and a side of the first liquid storage cavity 112 facing the distal end 120 is open. During use, the liquid substrate in the first liquid storage cavity 112 exits from the side of the distal end 120.

In some embodiments, the first liquid storage cavity 112 is mainly defined between the first part 11 of the first housing 10 and the aerosol output tube 111. In addition, the first housing 10 is transparent. In this way, the liquid substrate in the first liquid storage cavity 112 is visible through an outer surface of the first part 11 of the first housing 10.

As shown in FIG. 1 to FIG. 7, the first body 100 further includes:
a closing element 140, arranged substantially perpendicular to a longitudinal direction of the first body 100. The closing element 140 is arranged on the side of the first liquid storage cavity 112 facing the distal end 120 and is used for closing the side of the first liquid storage cavity 112 facing the distal end 120. A liquid output channel 143 is defined on the closing element 140, for providing a channel path for the liquid substrate in the first liquid storage cavity 112 to exit or to be output. After assembly, the liquid substrate in the first liquid storage cavity 112 can only be output or exit through the liquid output channel 143 of the closing element 140. The closing element 140 is rigid, for example, made of a rigid polymer plastic.

As shown in FIG. 1 to FIG. 7, the liquid output channel 143 extends substantially along the longitudinal direction of the first body 100. In addition, the liquid output channel 143 includes a first section 1431 and a second section 1432 that are sequentially arranged. The first section 1431 is close to and communicates with the first liquid storage cavity 112.

In an embodiment, a cross-sectional area or diameter of the first section 1431 is greater than a cross-sectional area or diameter of the second section 1432. For example, in some specific embodiments, the diameter of the first section 1431 is 2.5-4.0 mm; and the diameter of the second section 1432 is 1.5-3.0 mm.

As shown in FIG. 1 to FIG. 7, the closing element 140 is arranged in a substantially annular shape. The closing element 140 is arranged with a first convex edge 141 extending toward the proximal end 110. The first convex edge 141 surrounds and defines the first section 1431 of the liquid output channel 143. The first convex edge 141 further surrounds and defines a first air tube plug-in hole 142. During assembly, the aerosol output tube 111 passes through the first air tube plug-in hole 142 of the closing element 140. After passing through the closing element 140, the aerosol output tube 111 is at least partially located between the closing element 140 and the distal end 120.

As shown in FIG. 1 to FIG. 7, a cavity 150 is defined between the closing element 140 and the distal end 120. The aerosol output tube 111 extends into the cavity 150 after at least partially passing through the closing element 140.

As shown in FIG. 1 to FIG. 7, the closing element 140 is provided with at least one or more liquid output connectors 144 arranged extending toward the distal end 120. The at least one or more liquid output connectors 144 are substantially located in the cavity 150. The liquid output connector 144 is hollow and tubular, and the liquid output connector 144 surrounds or defines the second section 1432 of the liquid output channel 143 inside. When the first body 100 is combined with the second body 200, the liquid output connector 144 is inserted or extended into a liquid input interface 322 of the second body 200, to allow the second body 200 to be supplemented with the liquid substrate in the first liquid storage cavity 112. In some embodiments, an axial length of the liquid output connector 144 and/or the second section 1432 of the liquid output channel 143 is in a range of 4-8 mm. For example, in a specific embodiment, the axial length of the second section 1432 of the liquid output channel 143 is 4.8 mm. An inner diameter of the liquid output connector 144 is a diameter of the second section 1432, in a range of 1.5-3.0 mm.

As shown in FIG. 1 to FIG. 7, the liquid output connector 144 has a free end facing the distal end 120. Moreover, the free end of the liquid output connector 144 facing the distal end 120 is closed. A liquid outlet 145 is provided on a side wall of the liquid output connector 144, for the liquid substrate to flow out. In some embodiments, a diameter or a width of the liquid outlet 145 can restrict the liquid substrate from flowing out massively, and allow the liquid substrate to flow out only at a predetermined rate. In a specific embodiment, the diameter or the width of the liquid outlet 145 is in a range of 0.5-1.5 mm. In a more specific embodiment, the diameter or the width of the liquid outlet 145 is 0.8 mm. The liquid outlet 145 is arranged close to the free end of the liquid output connector 144.

As shown in FIG. 1 to FIG. 7, at least one or more capillary trenches 114 are arranged on an inner surface of the aerosol output tube 111. The capillary trenches 114 are arranged extending along a longitudinal direction. The capillary trench 114 has a width and/or a depth of approximately 0.2-1.0 mm. Further, during use, when the aerosol is delivered from the interior of the aerosol output tube 111 to the air outlet 113, the capillary trenches 114 can adsorb and retain, by means of a capillary action, an aerosol condensate on the inner surface of the aerosol output tube 111, which is beneficial for preventing the aerosol condensate from being smoked by the user.

As shown in FIG. 1 to FIG. 7, the first body 100 further includes:
a flexible first sealing element 130, at least partially mounted or arranged between the first housing 10/the aerosol output tube 111 and the closing element 140, for providing sealing therebetween. The flexible first sealing element 130 is made of, for example, flexible silicone, a thermoplastic elastomer, or other materials.

Specifically, a first avoidance hole 131 and a second avoidance hole 132 are arranged on the first sealing element 130. During assembly, the first avoidance hole 131 is opposite to the first air tube plug-in hole 142 of the closing element 140, thus allowing the aerosol output tube 111 to pass through the first avoidance hole 131 to be assembled to the first air tube plug-in hole 142. Moreover, the second avoidance hole 132 is disposed opposite to the liquid output channel 143 of the closing element 140, thus allowing the liquid substrate in the first liquid storage cavity 112 to enter the liquid output channel 143 through the second avoidance hole 132.

As shown in FIG. 1 to FIG. 7, the flexible first sealing element 130 is substantially in a sheet shape arranged perpendicular to the longitudinal direction of the first body 100. In addition, the first sealing element 130 at least partially surrounds or encloses the closing element 140.

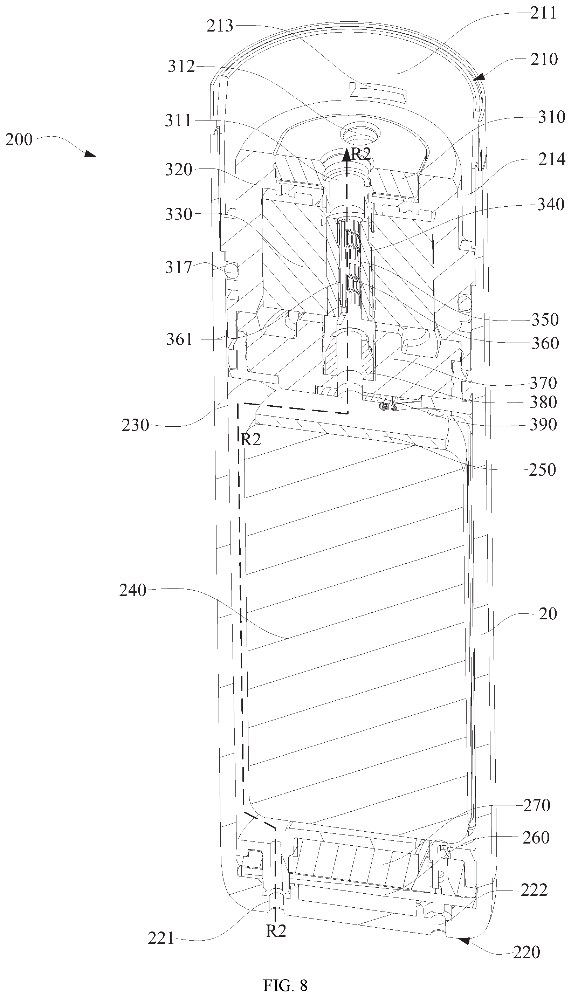

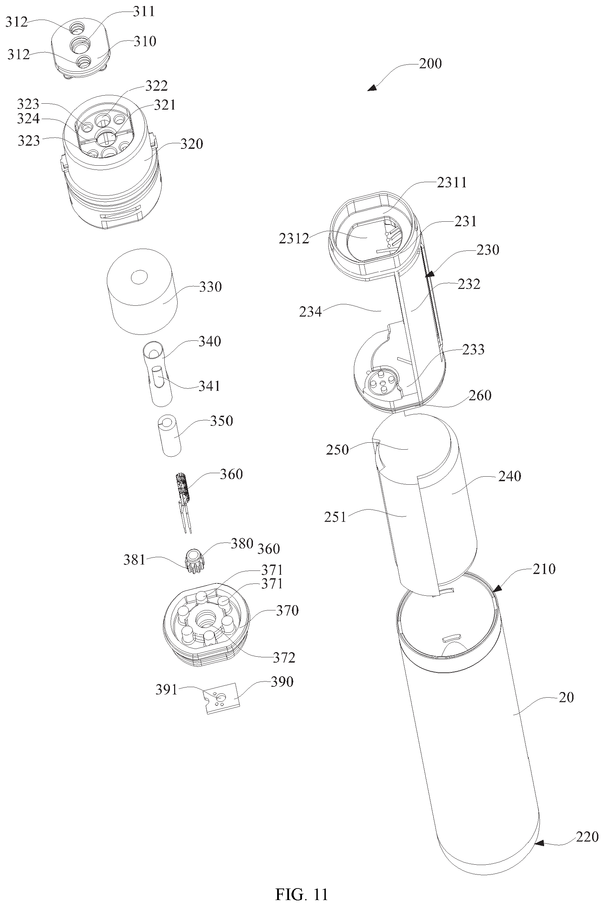

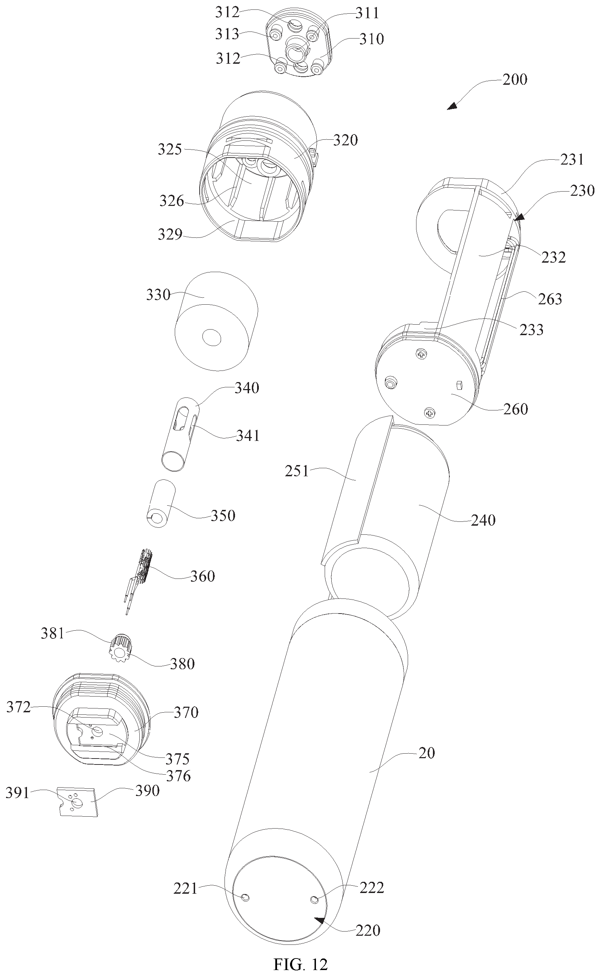

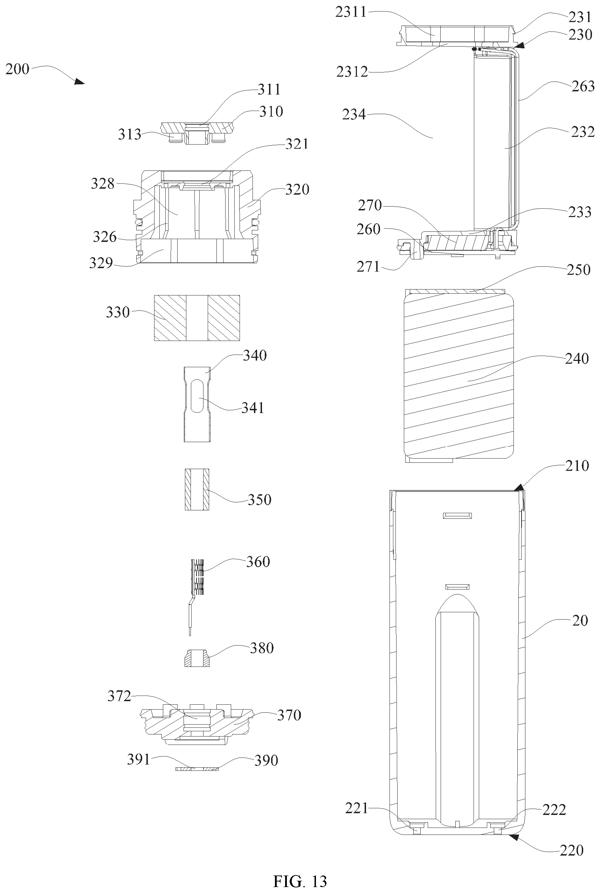

As shown in FIG. 8 to FIG. 21, the second body 200 includes:
a first end 210 and a second end 220 facing away from each other along the longitudinal direction; and
a second housing 20 extending between the first end 210 and the second end 220.

The second housing 20 is open at the first end 210 of the second body 200, thus allowing the second body 200 to define a receiving cavity 211 at the first end 210. When the first body 100 is combined with the second body 200, the receiving cavity 211 is used for receiving a part of the first body 100. Specifically, the second part 12 of the first housing 10 is inserted into or received in the receiving cavity 211.

As shown in FIG. 8 to FIG. 21, the second body 200 further includes:
an atomization module 300, including a plurality of functional components that jointly define atomization of a liquid substrate, where the atomization module is located in the second housing 20 and is close to or defines the receiving cavity 211;
a cell 240, configured to supply power, where after being assembled, the cell 240 is located between the atomization module and the second end 220; and
a control circuit board 260, located between the cell 240 and the second end 220, where the control circuit board 260 is arranged substantially perpendicular to a longitudinal direction of the second body 200; and a circuit is arranged on the control circuit board 260, and is configured to control the cell 240 to supply power to the atomization module.

As shown in FIG. 8 to FIG. 21, the atomization module 300 includes:
a support element 320, configured to accommodate, support, or retain each of the functional components used for atomization of the liquid substrate, where the support element 320 is constructed in a cylindrical shape;
a tubular element 340, accommodated or held in the support element 320, where the tubular element 340 is arranged extending along a longitudinal direction of the support element 320;
a second liquid storage cavity 328, defined by a partial space between the support element 320 and the tubular element 340; or defined by a hollow part of the support element 320, where the second liquid storage cavity 328 is located in the support element 320; and the second liquid storage cavity 328 forms a liquid substrate storage space located in the second body 200, for storing a liquid substrate;
a liquid retaining element 330, made of a flexible or rigid porous material or a fiber material, for adsorbing and retaining the liquid substrate stored in the second liquid storage cavity 328, where the liquid retaining element 330 and/or the second liquid storage cavity 328 is substantially annular in shape; and
an atomization assembly, located in the tubular element 340, and in fluid communication with the liquid retaining element 330 and/or the second liquid storage cavity 328, for drawing and atomizing a liquid substrate to generate an aerosol. Referring to FIG. 8 to FIG. 21, the atomization assembly includes: a liquid guide element 350 and a heating element 360 combined with the liquid guide element 350.

In the foregoing embodiments, the components for the function of atomizing the liquid substrate are designed as a modular module as a whole, which is beneficial for preparation, assembly, or the like of each functional module of the second body 200.

In some embodiments, the support element 320 and/or the tubular element 340 is made of rigid ceramic, stainless steel, polymer plastic, or the like.

In some embodiments, the liquid retaining element 330 may be made of a rigid porous material such as porous ceramic or porous glass, or may be made of flexible porous fiber such as porous cotton fiber, porous non-woven fabric, or a porous sponge.

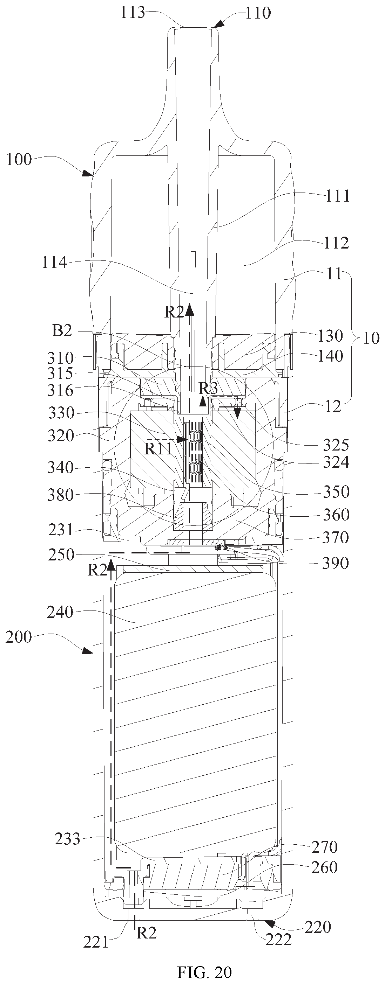

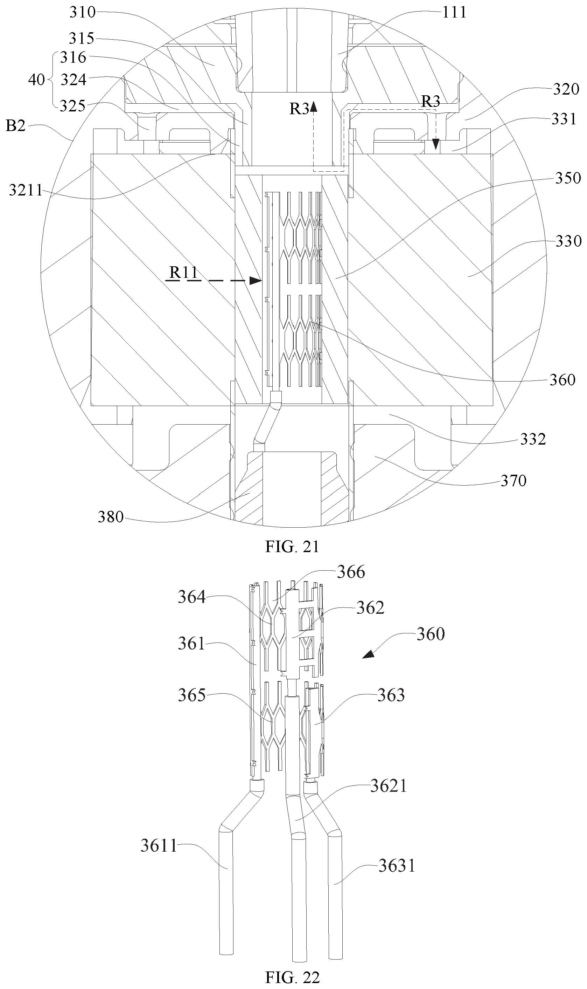

In some embodiments, the liquid guide element 350 is flexible in the embodiments, for example, made of flexible fiber such as cotton fiber, non-woven fabric, or a sponge. The liquid guide element 350 is configured to be tubular or cylindrical and arranged along a longitudinal direction of the support element 320. The liquid guide element 350 is coaxial with the liquid retaining element 330 and/or the tubular element 340, and is located inside the liquid retaining element 330 and/or the tubular element 340. Alternatively, in some other variant embodiments, the liquid guide element 350 may further include a rigid porous element, for example, porous ceramic or porous glass. An outer side surface of the liquid guide element 350 is in fluid communication with the liquid retaining element 330 and/or the second liquid storage cavity 328, so that the outer side surface of the liquid guide element 350 is used for drawing up the liquid substrate from the liquid retaining element 330 and/or the second liquid storage cavity 328, as shown by an arrow R11 in FIG. 20 and FIG. 21.

In some embodiments, the liquid guide element 350 is surrounded and retained by the liquid retaining element 330 and is in contact with the liquid retaining element 330 to form fluid communication. Alternatively, in some other embodiments, the liquid guide element 350 is retained in the tubular element 340, and the tubular element 340 is provided with a plurality of through holes 341. The liquid guide element 350 draws the liquid substrate from the liquid retaining element 330 and/or the second liquid storage cavity 328 through the through holes 341 in the tubular element 340.

In some embodiments, an inner surface of the liquid guide element 350 along a radial direction is configured as an atomization surface, and the atomization surface is combined with/attached to/abuts against the heating element 360. In this way, after being transferred to the atomization surface, the liquid substrate is heated by the heating element 360 and atomized to generate and release an aerosol. As shown in FIG. 8 to FIG. 21, the heating element 360 is arranged extending along a longitudinal direction of the liquid guide element 350. The heating element 360 is arranged coaxially with the liquid guide element 350. In some optional embodiments, the heating element 360 is a resistive heating mesh, a resistive heating coil, or the like. In this embodiment, the heating element 360 is a heating element that is rolled by using a sheet-like or mesh-like substrate. A conductive pin is soldered or arranged on the heating element 360, for guiding a current on the heating element 360.

In some other variant implementations, the heating element 360 may be combined with the liquid guide element 350 in a manner of printing, deposition, sintering, physical assembly, or the like. In some other variant implementations, the liquid guide element 350 may have a plane or a curved surface for supporting the heating element 360, and the heating element 360 is formed on the plane or the curved surface of the liquid guide element 350 in a manner of surface-mounting, printing, deposition, or the like. Alternatively, in some other variant implementations, the heating element 360 is a conductive trajectory formed on a surface of the liquid guide element 350. In some other variant implementations, a conductive trajectory of the heating element 360 may be in a form of a printed circuit formed by printing. In some other variant implementations, the heating element 360 is a patterned conductive trajectory. In some other implementations, the heating element 360 is planar. In some other variant implementations, the heating element 360 is a conductive trajectory extending in a circuitous, meandering, reciprocal, or zigzagging manner.

As shown in FIG. 8 to FIG. 21, a side of the support element 320 facing the first end 210 is closed. Moreover, a side of the support element 320 facing the second end 220 is open. Moreover, after assembly, the receiving cavity 211 for receiving a part of the first body 100 is defined between the support element 320 and the first end 210 of the second housing 20.

As shown in FIG. 8 to FIG. 10, the support element 320 is firmly connected to the second housing 20 by riveting, snapping, or the like. A sealing ring 317, such as an O-ring, surrounding the support element 320 is further arranged between the support element 320 and the second housing 20, for providing sealing between the support element 320 and the second housing 20. In some specific embodiments, a mounting groove that surrounds the support element 320 along a circumferential direction is arranged on an outer side surface of the support element 320. The sealing ring 317, such as an O-ring, is mounted or held in the mounting groove.

As shown in FIG. 8 to FIG. 10, an insertion gap 214 is further defined between the support element 320 and the second housing 20. The insertion gap 214 communicates with the receiving cavity 211. When the first body 100 is combined with the second body 200, the second part 12 of the first housing 10 of the first body 100 extends into the insertion gap 214 after passing through the receiving cavity 211. Further, when the first body 100 is combined with the second body 200, the atomization module 300 and/or the support element 320 at least partially extends into or is accommodated in the cavity 150 of the first body 100.

As shown in FIG. 8 to FIG. 21, the atomization module 300 further includes:
a flexible second sealing element 310, at least partially exposed to the receiving cavity 211. The second sealing element 310 is at least partially accommodated and held in the support element 320. The second sealing element 310 is made of flexible silicone, or a thermoplastic elastomer, or the like. When the first body 100 is received in or combined with the second body 200, the second sealing element 310 is connected to the aerosol output tube 111, and provides sealing between the support element 320 and the aerosol output tube 111. Moreover, when the first body 100 is received in or combined with the second body 200, the second sealing element 310 is connected to the liquid output connector 144 of the closing element 140, and provides sealing between the support element 320 and the liquid output connector 144.

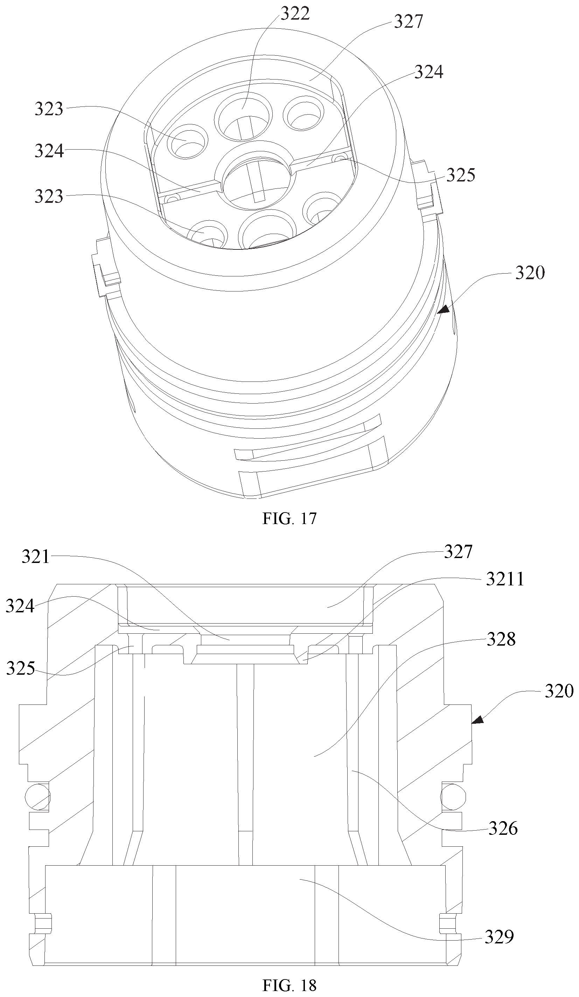

As shown in FIG. 17 and FIG. 18, a first accommodating cavity 327 is provided on the side of the support element 320 facing the first end 210, for accommodating and mounting the second sealing element 310. When the second sealing element 310 is accommodated or mounted in the first accommodating cavity 327, a surface of the second sealing element 310 is substantially flush with a surface of the support element 320.

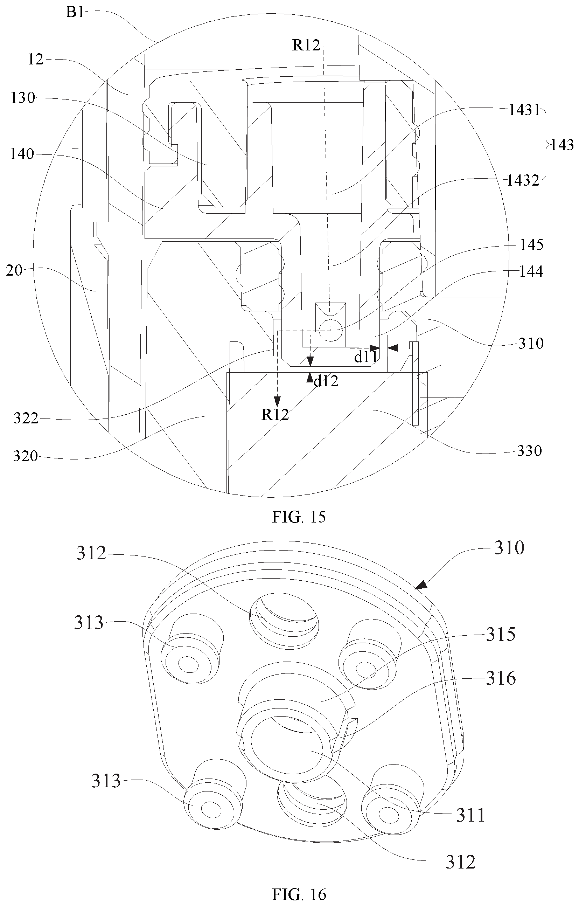

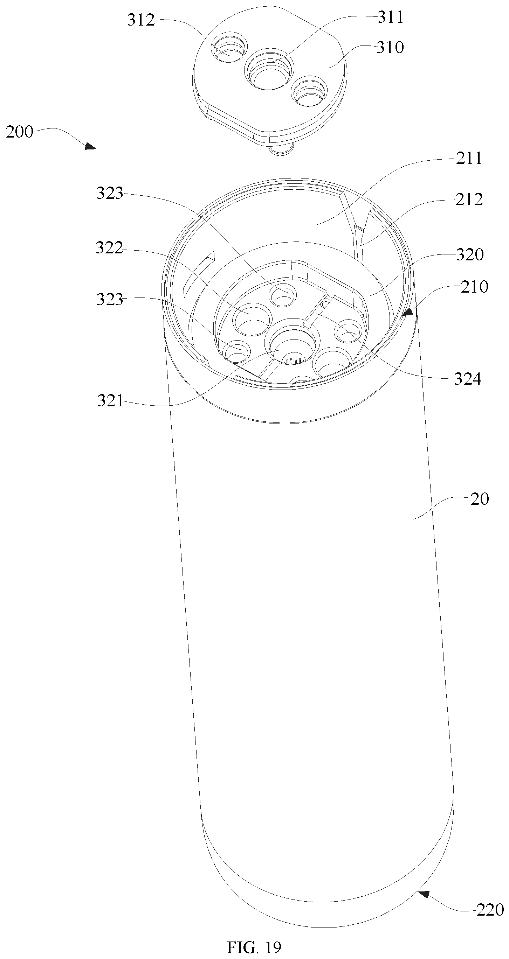

As shown in FIG. 8 to FIG. 21, the support element 320 is further provided with liquid injection holes 323 running from the first accommodating cavity 327 to the second liquid storage cavity 328. The liquid injection hole 323 is used for injecting a liquid substrate into the second liquid storage cavity 328 by an injection device such as an injector through the liquid injection hole 323. As shown in FIG. 16 to FIG. 19, there are four liquid injection holes 323, which are arranged discretely. Correspondingly, a plurality of plugs 313 are arranged on the second sealing element 310. When the second sealing element 310 is mounted or accommodated in the first accommodating cavity 327 of the support element 320, each of the plurality of plugs 313 separately extends into each of the liquid injection holes 323, to close and plug the liquid injection holes 323. As shown in FIG. 19, during production, when a liquid substrate needs to be injected into the second liquid storage cavity 328, a user can remove the second sealing element 310 from the support element 320, to open and expose the liquid injection holes 323 to perform a liquid injection operation. Moreover, after completing the liquid injection operation, the user assembles the second sealing element 310 to the support element 320, to shield and plug the liquid injection holes 323.

As shown in FIG. 8 to FIG. 21, the support element 320 is further provided with a connection hole 321. An upper end of the tubular element 340 facing the first end 210 is held in the connection hole 321 by means of riveting, interference, fastening, or the like. Moreover, the second sealing element 310 is provided with a second air tube plug-in hole 311 opposite to the connection hole 321. Correspondingly, when the first body 100 is combined with the second body 200, the aerosol output tube 111 of the first body 100 at least partially passes through the second air tube plug-in hole 311 of the second sealing element 310 and then extends into the connection hole 321 of the support element 320, to form an airflow connection. Moreover, when the first body 100 is combined with the second body 200, the second sealing element 310 at least partially and elastically provides sealing between the aerosol output tube 111 and the connection hole 321 of the support element 320.

As shown in FIG. 8 to FIG. 21, a liquid input interface 322 is further provided in the support element 320. The liquid input interface 322 extends from the first accommodating cavity 327 to the second liquid storage cavity 328. The second sealing element 310 is provided with a third avoidance hole 312 corresponding to the liquid input interface 322. When the second sealing element 310 is accommodated and mounted on the support element 320, the third avoidance hole 312 is opposite to the liquid input interface 322, so that the liquid input interface 322 is exposed and open.

As shown in FIG. 8 to FIG. 21, the atomization module 300 further includes:
a base 370, made of a material such as flexible silicone or a thermoplastic elastomer. The base 370 is combined with an opening of the support element 320 facing the second end 220, and is configured to close the opening of the support element 320 facing the second end 220.

As shown in FIG. 8 to FIG. 21, the support element 320 has a second accommodating cavity 329 on a second side facing the second end 220. After assembly, the base 370 at least partially extends into or is accommodated in the second accommodating cavity 329, and the base 370 and the support element 320 jointly defines the second liquid storage cavity 328. Alternatively, when the base 370 is at least partially assembled and accommodated in the second accommodating cavity 329, the flexible base 370 is at least partially pressed or compressed. This allows the base 370 to be used for closing or sealing the second liquid storage cavity 328 on the second side of the support element 320.

As shown in FIG. 8 to FIG. 21, the base 370 is substantially annular and has a through hole 372 running through the base 370 along an axial direction. During assembly, the tubular element 340 is at least partially inserted into the through hole 372, to be assembled with the base 370. After assembly, the tubular element 340 runs through the liquid retaining element 330. Moreover, after assembly, the tubular element 340 extends from the connection hole 321 of the support element 320 into the through hole 372 of the base 370.

As shown in FIG. 8 to FIG. 21, the atomization module 300 further includes:
an electrical connection element 390, configured to provide an electrical connection between the control circuit board 260 and the heating element 360. Specifically, the electrical connection element 390 is in a sheet-like or board-like shape. The electrical connection element 390 is arranged perpendicular to the longitudinal direction of the second body 200.

In some embodiments, the electrical connection element 390 includes a conductive sheet, or includes a conductive material coated or combined on a surface of an electrically insulating substrate. In some other specific embodiments, the electrical connection element 390 includes a circuit board such as a PCB board or an FPC board. After assembly, the heating element 360 and the control circuit board 260 are both connected to the electrical connection element 390, thus establishing an electrical connection therebetween.

As shown in FIG. 8 to FIG. 22, the heating element 360 is constructed to be in a cylindrical shape extending along the longitudinal direction of the liquid guide element 350. In this embodiment, the heating element 360 is a heating element that is rolled by using a sheet-like or mesh-like substrate. The rolled heating element 360 is a non-closed tube in a circumferential direction, and has a cylindrical shape with a side opening 366 extending from one end to the other end along a longitudinal direction.

As shown in FIG. 22, in this embodiment, the heating element 360 includes:
a first heating portion 364 and a second heating portion 365 spaced apart along a longitudinal direction, where the first heating portion 364 and the second heating portion 365 are each in a mesh shape with meshes;
a first electrode part 361, extending from one end of the heating element 360 to the other end, where moreover, the first electrode part 361 is located on a first side of the side opening 366; and
a second electrode part 362 and a third electrode part 363, spaced apart along a longitudinal direction and/or a circumferential direction of the heating element 360, where the second electrode part 362 and the third electrode part 363 are located on a second side of the side opening 366.

The first electrode part 361, the second electrode part 362, and the third electrode part 363 are dense. The first heating portion 364 is electrically connected between the first electrode part 361 and the second electrode part 362. The second heating portion 365 is electrically connected between the first electrode part 361 and the third electrode part 363.

As shown in FIG. 22, the heating element 360 further includes:
a first conductive lead 3611, connected to the first electrode part 361 in a manner such as soldering;
a second conductive lead 3621, connected to the second electrode part 362 in a manner such as soldering; and
a third conductive lead 3631, connected to the third electrode part 363 in a manner such as soldering.

In terms of electrical connection, the electrical connection element 390 in the sheet-like shape has an upper surface and a lower surface that are opposite to each other. After passing through the through hole 372 of the base 370, the first conductive lead 3611, the second conductive lead 3621, and the third conductive lead 3631 of the heating element 360 are connected to the upper surface of the electrical connection element 390. Moreover, the lower surface of the electrical connection element 390 is connected to the control circuit board 260 by soldering a conductive lead 263. Therefore, during control, the control circuit board 260 can selectively connect the first electrode part 361, the second electrode part 362, and the third electrode part 363 to positive and negative terminals of the cell 240 in different connection manners, to selectively control one of the first heating portion 364 and the second heating portion 365 to heat separately, or control both of the first heating portion 364 and the second heating portion 365 to heat simultaneously in series or parallel. For example, in a specific embodiment, the control circuit board 260 electrically connects the first electrode part 361 to the positive terminal of the cell 240 and electrically connects the second electrode part 362 and the third electrode part 363 to the negative terminal of the cell 240, to control the first heating portion 364 and the second heating portion 365 to heat simultaneously in parallel.

As shown in FIG. 8 to FIG. 22, the atomization module 300 further includes:
a wire separation element 380, mounted or arranged in the through hole 372 of the base 370 and located between the heating element 360 and the electrical connection element 390. Alternatively, the wire separation element 380 is mounted or arranged in the tubular element 340 and located between the heating element 360 and the electrical connection element 390. The wire separation element 380 is annular in shape, and a plurality of ridged projections 381 spaced apart along a circumferential direction are arranged on an outer side surface of the wire separation element 380. During assembly, the first conductive lead 3611, the second conductive lead 3621, and the third conductive lead 3631 are respectively restricted in gaps between the plurality of ridged projections 381 to form isolation, thereby preventing the first conductive lead 3611, the second conductive lead 3621, and the third conductive lead 3631 from abutting against or coming into contact with each other during assembly to cause a problem such as a short circuit.

As shown in FIG. 8 to FIG. 21, a surface of the base 370 facing the second end 220 is provided with a retaining groove 375, and the electrical connection element 390 is mounted in the retaining groove 375 and is limited and retained by clamping hooks 376 on two sides of the retaining groove 375.

As shown in FIG. 8 to FIG. 23, the second body 200 further includes:
a support 230, located between the atomization module 300 and the control circuit board 260. The support 230 includes a first support part 231, a second support part 232, and a third support part 233 sequentially arranged along a longitudinal direction.

The first support part 231 at least partially supports and retains the atomization module 300. Specifically, the first support part 231 is substantially annular and defines a retaining cavity 2311. After assembly, the atomization module 300 is at least partially accommodated and mounted in the retaining cavity 2311.

The second support part 232 defines a cell chamber 234 located between the first support part 231 and the second support part 233, for at least partially supporting and retaining the cell 240.

The third support part 233 at least partially supports and retains the control circuit board 260. Specifically, for example, the control circuit board 260 is connected to the third support part 233 by using a fastener such as a screw.

As shown in FIG. 8 to FIG. 23, a surface of the control circuit board 260 facing the cell 240 is further provided with:
an airflow sensor 290, such as a microphone sensor or a MEMS sensor, configured to sense a change in an airflow flowing through the airflow channel during inhalation. The control circuit board 260 controls, according to a sensing result of the airflow sensor 290, to supply power to the heating element 360.

As shown in FIG. 8 to FIG. 23, a surface of the control circuit board 260 facing the cell 240 is further provided with:
an electrical connection portion 261, which is, for example, a pad or a solder joint arranged on the surface of the control circuit board 260. In this embodiment, the conductive lead 263 is connected to the electrical connection portion 261 in a manner such as soldering, thus electrically connecting the control circuit board 260 and the electrical connection element 390. Moreover, a positive tab and a negative tab of the cell 240 are soldered to the electrical connection portion 261, thus allowing the control circuit board 260 to be connected to the cell 240.

As shown in FIG. 8 to FIG. 23, the second body 200 further includes:
a flexible wrapping element 270, which is made of, for example, flexible silicone. The wrapping element 270 is at least partially mounted and arranged between the third support part 233 of the support 230 and the control circuit board 260. A sensor accommodating portion 272 is arranged on the wrapping element 270. The sensor accommodating portion 272 is mainly configured to wrap the airflow sensor 290 and isolate two opposite sensing surfaces of the airflow sensor 290.

The wrapping element 270 is further provided with a first wire avoidance hole 271, arranged opposite to the electrical connection portion 261 on the control circuit board 260. The third support part 233 of the support 230 is provided with a second wire avoidance hole 235. After assembly, the conductive lead 263 passes through the first wire avoidance hole 271 and the second wire avoidance hole 235 and then is connected to the electrical connection portion 261.

As shown in FIG. 8 to FIG. 21, the electronic atomization device further defines an airflow channel for outputting, during inhalation, the aerosol generated by the atomization module 300 to the air outlet 113, for example, as shown by an arrow R2 in FIG. 8 to FIG. 21. The airflow channel of the electronic atomization device has one part defined by the aerosol output tube 111 of the first body 100 and one part defined by the second body 200. In addition, the airflow channel runs through the atomization module 300.

As shown in FIG. 8 to FIG. 23, related construction content of a complete airflow channel includes:
an air inlet 221, arranged at the second end 220 of the second housing 20, for allowing external air to enter the second housing 20 during inhalation.

The control circuit board 260 is provided with a first air hole 262.

The wrapping element 270 is provided with a second air hole 273.

The third support part 233 of the support 230 is provided with a third air hole 234.

The first support part 231 of the support 230 defines a central hole 2312 therein.

The electrical connection element 390 is provided with a fourth air hole 391.

A complete airflow path during inhalation is shown by the arrow R2 in FIG. 8 to FIG. 23. The external air entering from the air inlet 221 sequentially passes through the first air hole 262 of the control circuit board 260, the second air hole 273 of the wrapping element 270, and the third air hole 234 of the third support part 233, then flows into the central hole 2312 of the first support part 231 through a gap between the cell 240 and the second housing 20, then passes through the fourth air hole 391 in the electrical connection element 390, the through hole 372 in the base 370, and the wire separation element 380, and is then delivered to the heating element 360; and then passes through the heating element 360, carries the aerosol generated by heating to sequentially pass through the connection hole 321 and the aerosol output tube 111, and is output at the air outlet 113 to be inhaled by a user.

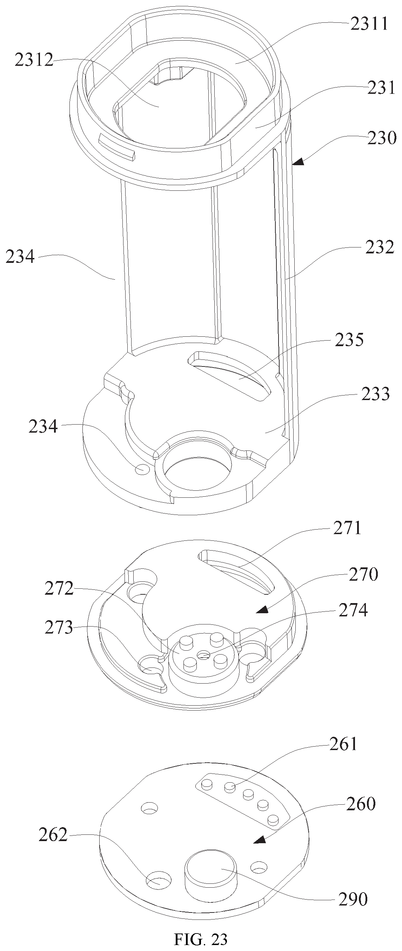

As shown in FIG. 8 and FIG. 23, the second end 220 of the second housing 20 is further provided with:
a sensor sensing hole 222, for allowing a second sensing surface of the airflow sensor 290 to communicate with the external atmosphere. Specifically, after assembly, a first sensing surface of the airflow sensor 290 facing the cell 240 is partially covered by the sensor accommodating portion 272 of the wrapping element 270, and communicates with the airflow channel through a sensing connection hole 274 in the sensor accommodating portion 272. The second sensing surface of the airflow sensor 290 facing the control circuit board 260 communicates with the external atmosphere through the air hole in the control circuit board 260 and the sensor sensing hole 222. Moreover, the airflow sensor 290 determines inhalation by the user according to a difference between pressure values sensed by the first sensing surface and the second sensing surface.

As shown in FIG. 8 to FIG. 23, the second body 200 further includes:
an absorbing element 250, made of a flexible porous material, such as cotton fiber or a sponge. The absorbing element 250 is at least partially located between the cell 240 and the first support part 231 of the support 230 and is arranged opposite to the central hole 2312 in the first support part 231. During use, the absorbing element 250 is configured to absorb an aerosol condensate that falls toward the cell 240 in the airflow channel. The absorbing element 250 may further have a plurality of fin portions 251 that surround the cell 240 along a circumferential direction. The plurality of fin portions 251 absorb the aerosol condensate between the cell 240 and the second housing 20.

As shown in FIG. 8 to FIG. 21, an inner side surface of the support element 320 is provided with a plurality of convex ridges 326 extending along the longitudinal direction. The plurality of convex ridges 326 are spaced apart along the circumferential direction of the support element 320. When the liquid retaining element 330 is mounted in the support element 320, an outer side surface of the liquid retaining element 330 abuts against the convex ridges 326. Therefore, a plurality of gaps located between adjacent ones of the convex ridges 326 are defined between the outer side surface of the liquid retaining element 330 and the inner side surface of the support element 320.

As shown in FIG. 8 to FIG. 21, a second convex edge 3211 extending from the connection hole 321 toward the second liquid storage cavity 328 along the longitudinal direction is arranged in the support element 320. The second convex edge 3211 is annular. The second convex edge 3211 at least partially surrounds and defines the connection hole 321. A plurality of support protrusions 371 are arranged on a surface of the base 370 facing the second liquid storage cavity 328. After assembly, the annular liquid retaining element 330 is longitudinally retained between the support element 320 and the base 370, and specifically is longitudinally clamped or retained between the second convex edge 3211 and the support protrusion 371. Specifically, after assembly, the liquid retaining element 330 has an upper surface abutting against the second convex edge 3211 and/or the plurality of plugs 313 of the second sealing element 310, and a lower surface abutting against the support protrusion 371. Therefore, after assembly, in a longitudinal direction of the atomization module 300, a first compartment 331 is defined between the liquid retaining element 330 and the support element 320; and a second compartment 332 is defined between the liquid retaining element 330 and the base 370. Moreover, after assembly, the first compartment 331 and the second compartment 332 are in communication with each other through the gaps between the adjacent ones of the convex ridges 326. Alternatively, the second liquid storage cavity 328 has a first inner wall close to the first end 210, and the first inner wall is defined by the support element 320. After assembly, a first compartment 331 is formed between an upper surface of the liquid retaining element 330 and the first inner wall. Alternatively, the second liquid storage cavity 328 has a second inner wall close to the first end 210, and the second inner wall is defined by the base 370. After assembly, a second compartment 332 is formed between a lower surface of the liquid retaining element 330 and the second inner wall. Alternatively, the second liquid storage cavity 328 has an inner side wall surrounding the second liquid storage cavity 328, and the inner side wall is defined by an inner side surface of the second liquid storage cavity 328. The convex ridges 326 are located on the inner side wall of the second liquid storage cavity 328, and a gap between the convex ridges 326 on the inner side wall provides fluid communication between the first compartment 331 and the second compartment 332.

In some embodiments, the liquid retaining element 330 is defined by a single porous fiber element. Alternatively, in some other variant embodiments, the liquid retaining element 330 includes a first porous fiber material layer and a second porous fiber material layer sequentially arranged along an axial direction. In addition, the first porous fiber material layer defines the upper surface of the liquid retaining element 330; and the second porous fiber material layer defines the lower surface of the liquid retaining element 330. Moreover, the first porous fiber material layer and the second porous fiber material layer are stacked to form the liquid retaining element 330.

In some embodiments, the second porous fiber material layer is made of a flexible capillary fiber material, such as natural cotton fiber or non-woven fabric fiber.

In some embodiments, the first porous fiber material layer includes artificial cotton, or hard artificial cotton or artificial foam made of filamentous polyurethane. For example, the first porous fiber material layer uses 138# hard synthetic organic polymer fiber. For another example, the first porous fiber material layer uses 138# hard synthetic organic polymer fiber, with a density of 0.1-0.9 mg/mm3. The first porous fiber material layer is made of oriented fiber oriented and aligned substantially along a length direction, a width direction, or a radial direction. The arrangement of the oriented fiber in the length direction or the width direction of the first porous fiber material layer allows the first porous fiber material layer to exhibit a relatively strong bending resistance and thus present a hardness feature. Specifically, for example, the first porous fiber material layer is hard artificial cotton including oriented polyester fiber, or hard artificial cotton or artificial foam made of filamentous polyurethane.

As shown in FIG. 8 to FIG. 21, after assembly, the upper surface of the liquid retaining element 330 abuts against a port of the liquid input interface 322 defined by the support element 320, to prevent air in the liquid input interface 322 from communicating with external air through the first compartment 331 and an air exchange channel 40. When the liquid substrate in the second liquid storage cavity 328 is consumed, a part that is in the liquid retaining element 330 and that is close to the port of the liquid input interface 322 is free of the liquid substrate. Porous pores in this part provide a channel for transmitting negative pressure between a capillary channel/the liquid input interface 322 and the second liquid storage cavity 328, allowing the capillary channel/the liquid input interface 322 to maintain balance with the negative pressure or pressure in the second liquid storage cavity 328.

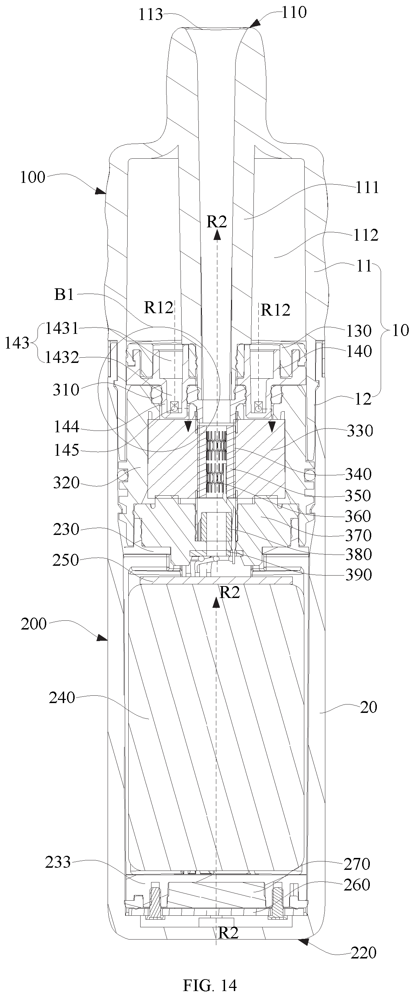

As shown in FIG. 14 and FIG. 15, when the first body 100 is combined with the second body 200, the liquid output connector 144 of the closing element 140 of the first body 100 at least partially extends into or penetrates the liquid input interface 322, thus connecting the first liquid storage cavity 112 of the first body 100 and the second liquid storage cavity 328 of the second body 200 in fluid communication.

As shown in FIG. 14 and FIG. 15, when the first body 100 is combined with the second body 200, the liquid outlet 145 in the liquid output connector 144 of the closing element 140 is located in the liquid input interface 322. Moreover, an outer side surface of the liquid output connector 144 is not in contact with an inner surface of the liquid input interface 322, so that a first distance d11 is defined between the outer side surface of the liquid output connector 144 and the inner surface of the liquid input interface 322. Moreover, the free end of the liquid output connector 144 inserted into the liquid input interface 322 is not in contact with the liquid retaining element 330 located in the second liquid storage cavity 328, so that a second distance d12 is defined between the liquid output connector 144 and the liquid retaining element 330.

In this embodiment, the first distance d11 and/or the second distance d12 is approximately in a range of 0.1-1.5 mm. For example, in a specific embodiment, the first distance d11 and/or the second distance d12 is 0.25 mm. During use, the first distance d11 defines the capillary channel located between the liquid output connector 144 and the liquid input interface 322. In addition, the liquid substrate flowing out from the liquid outlet 145 in the liquid output connector 144 is adsorbed and retained by the capillary channel defined by the first distance d11, to prevent the liquid substrate in the first liquid storage cavity 112 from being massively added to the second liquid storage cavity 328.

During use, the capillary channel defined by the first distance d11 can control, by means of a capillary action, the liquid substrate in the first liquid storage cavity 112 to be added to the second liquid storage cavity 328 in a predetermined amount. This is beneficial for preventing oversaturated storage of the liquid substrate in the second liquid storage cavity 328.

Specifically, during use, when an amount of the liquid substrate adsorbed and retained by the liquid retaining element 330 in the second liquid storage cavity 328 is relatively sufficient, negative pressure or pressure in the second liquid storage cavity 328 and/or the first compartment 331 is lower than a preset threshold. In this case, the liquid substrate flowing out from the liquid outlet 145 in the liquid output connector 144 is adsorbed and retained in the capillary channel defined by the first distance d11 and forms a liquid film to close the liquid outlet 145, thereby preventing the liquid substrate in the first liquid storage cavity 112 from being massively added to the second liquid storage cavity 328. However, when the liquid substrate adsorbed and retained by the liquid retaining element 330 in the second liquid storage cavity 328 and/or the first compartment 331 is consumed, causing the negative pressure in the second liquid storage cavity 328 and/or the first compartment 331 to exceed a predetermined threshold, driven by the negative pressure, the liquid substrate retained in the capillary channel defined by the first distance d11 flows into and is added to the second liquid storage cavity 328 along an inner surface of the liquid input interface 322, as shown by an arrow R12 in FIG. 14 and FIG. 15. When a predetermined amount of the liquid substrate is added to the second liquid storage cavity 328, causing the negative pressure in the second liquid storage cavity 328 and/or the first compartment 331 to be lower than the predetermined threshold again, the liquid substrate flowing out from the liquid outlet 145 in the liquid output connector 144 is adsorbed by the capillary channel defined by the first distance d11, thus achieving balance and preventing the liquid substrate from being continuously added to the second liquid storage cavity 328. In this way, the retaining of the capillary channel defined by the first distance d11 between the liquid output connector 144 and the liquid input interface 322 can control, with inhalation or use by the user, the first liquid storage cavity 112 to supplement the second liquid storage cavity 328 with only the predetermined amount of the liquid substrate each time.

Alternatively, in some other variant embodiments, the capillary channel defined by the first distance d11 may be variably replaced with a capillary groove on the outer side surface of the liquid output connector 144 and/or the inner surface of the liquid input interface 322. In this variant embodiment, when the liquid output connector 144 is inserted into the liquid input interface 322, the outer side surface of the liquid output connector 144 may abut against or may be in contact with the inner surface of the liquid input interface 322, and the capillary groove on the outer side surface of the liquid output connector 144 and/or the inner surface of the liquid input interface 322 defines and forms a capillary channel therebetween.

Alternatively, in some other variant embodiments, a plurality of convex ridges extending along a longitudinal direction are arranged on the outer side surface of the liquid output connector 144. Moreover, the plurality of convex ridges are spaced apart around the liquid output connector 144 in a circumferential direction. When the liquid output connector 144 is inserted into the liquid input interface 322, gaps between the plurality of convex ridges define and form capillary channels located between the liquid output connector 144 and the liquid input interface 322.

As shown in FIG. 14 and FIG. 15, the second distance d12 defined between the liquid output connector 144 and the liquid retaining element 330 can prevent the liquid substrate in the capillary channel defined by the first distance d11 from being absorbed by the porous liquid retaining element 330.

In an embodiment, a diameter of the second section 1432 of the liquid output channel 143 defined in the liquid output connector 144 is greater than a diameter of the liquid outlet 145 on the side wall of the liquid output connector 144. Moreover, the diameter of the liquid outlet 145 is greater than the first distance d11. Such an arrangement gradually reduces an area of a channel during an outflow of the liquid substrate, which is beneficial for gradually reducing a flow rate and improving liquid retaining to supply a predetermined amount of the liquid substrate.

As shown in FIG. 3 to FIG. 15, there may be a plurality of liquid output connectors 144 of the first body 100. For example, in this embodiment, there are two liquid output connectors 144 of the first body 100. Moreover, there may be a plurality of liquid outlets 145 in each liquid output connector 144. During use, when the liquid substrate in the first liquid storage cavity 112 flows out from at least one of the liquid outlets 145 in the plurality of liquid output connectors 144, at least another liquid outlet 145 is used as an air inlet for providing air to enter the liquid output connector 144 and then into the first liquid storage cavity 112, to maintain pressure balance in the first liquid storage cavity 112.

As shown FIG. 3 to FIG. 15, there are at least two liquid output connectors 144 of the first body 100. For example, the liquid output connectors 144 may include a first connector and a second connector arranged in parallel. Correspondingly, there are also at least two liquid input interfaces 322 on the second body 200. For example, the liquid input interfaces 322 may include a first interface and a second interface arranged in parallel. The first connector and the second connector are respectively arranged on two sides of the atomization assembly, and the first interface and the second interface are respectively arranged on two sides of the atomization assembly. In some embodiments, a free end of the first connector is flush with a free end of the second connector. Moreover, a distance between a liquid outlet 145 in the first connector and the free end of the first connector is equal to a distance between a liquid outlet 145 in the second connector and the free end of the second connector.

For example, in an embodiment, a side wall of each liquid output connector 144 is provided with two liquid outlets 145 that are opposite to each other along a radial direction. When the liquid substrate in the liquid output connector 144 flows out from one of the two liquid outlets 145, air in the second liquid storage cavity 328 and/or the first compartment 331 enters the liquid output connector 144 from the other of the two liquid outlets 145.

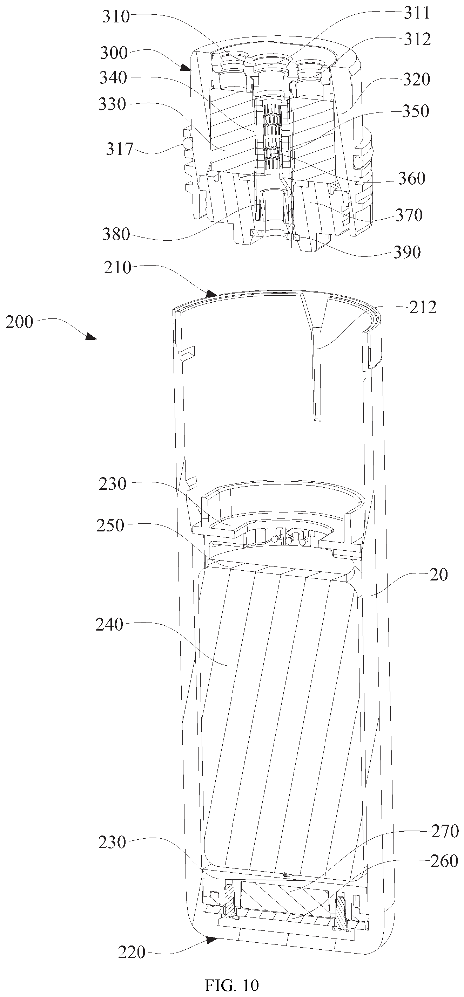

As shown by an arrow R3 in FIG. 10 to FIG. 21, the atomization module 300 further defines an air exchange channel 40, for allowing the second liquid storage cavity 328 and/or the first compartment 331 to communicate with an airflow channel running through the atomization module 300. The air exchange channel 40 is used for balancing pressure in the second liquid storage cavity 328 and/or the first compartment 331. For example, when negative pressure in the second liquid storage cavity 328 and/or the first compartment 331 exceeds a predetermined threshold, the air exchange channel 40 provides a channel path for air to enter the second liquid storage cavity 328 and/or the first compartment 331, to relieve the pressure in the second liquid storage cavity 328 and/or the first compartment 331. For another example, when the internal pressure exceeds the predetermined threshold due to oversaturated adsorption of the liquid substrate by the liquid retaining element 330 of the second liquid storage cavity 328, the air in the second liquid storage cavity 328 and/or the first compartment 331 is discharged to the outside through the air exchange channel 40 to relieve the pressure in the second liquid storage cavity 328 and/or the first compartment 331.

As shown by an arrow R3 in FIG. 10 to FIG. 21, the air exchange channel 40 is formed or defined between the support element 320 and the second sealing element 310.

The air exchange channel 40 includes a first vent slot 316 provided on an outer side surface of an extension wall 315. Specifically, the second sealing element 310 is provided with the extension wall 315 at least partially extending from the connection hole 321 of the support element 320 into the tubular element 340. In addition, when the extension wall 315 partially extends into the tubular element 340, the extension wall 315 and the liquid guide element 350 are spaced apart, for example, with a distance of 0.2-2.0 mm therebetween. Moreover, the extension wall 315 is annular in shape, partially extending from the connection hole 321 of the support element 320 into the tubular element 340, and at least partially surrounding or defining an airflow channel running through the atomization module 300. After assembly, a gap is maintained between the extension wall 315 and the tubular element 340 by using the vent slot 316, so that the air exchange channel 40 communicates with the airflow channel by using the vent slot 316.

The air exchange channel 40 further includes a second vent slot 324 provided on a surface of the support element 320, and a vent hole 325 running from the second vent slot 324 to the second liquid storage cavity 328 and/or the first compartment 331. After assembly, the second vent slot 324 is at least partially opposite to, aligned with, or overlapped with the first vent slot 316, so that the second vent slot 324 is in communication with the first vent slot 316 after assembly. Therefore, finally, the vent hole 325, the second vent slot 324, and the first vent slot 316 jointly define the air exchange channel 40 that allows the second liquid storage cavity 328 and/or the first compartment 331 to communicate with the airflow channel.

In an embodiment, the second vent slot 324 is provided on a bottom wall of the first accommodating cavity 327. Moreover, the vent hole 325 and the first vent slot 316 are respectively connected to two sides of the second vent slot 324. Alternatively, the second vent slot 324 extends from the vent hole 325 to the first vent slot 316. Alternatively, in some other variant embodiments, the second vent slot 324 may be provided on a surface of the second sealing element 310 facing the support element 320.

In an embodiment, a diameter or a width of the air exchange channel 40 is in a range of 0.2-2.0 mm. For example, a diameter or a width of the vent hole 325/the second vent slot 324/the first vent slot 316 is in a range of 0.2-2.0 mm.

During use, with no inhalation by the user, the first distance d11 between the liquid output connector 144 of the first body 100 and the liquid input interface 322 of the second body 200 adsorbs the liquid substrate flowing out from the liquid outlet 145 and is in a balanced state with the pressure in the second liquid storage cavity 328. With inhalation by the user, as the liquid substrate is consumed and the negative pressure in the airflow channel increases, the negative pressure in the second liquid storage cavity 328 increases to exceed the predetermined threshold and thus breaks balance. The negative pressure in the second liquid storage cavity 328 can be transmitted to the liquid input interface 322 through a material gap of the liquid retaining element 330. In this case, the liquid substrate adsorbed by the first distance d11 flows downward into the second liquid storage cavity 328 and is absorbed by the liquid retaining element 330. Until the user stops the inhalation, air enters the second liquid storage cavity 328 from the air exchange channel 40, and an amount of the liquid substrate added to the second liquid storage cavity 328 jointly relieves the negative pressure in the second liquid storage cavity 328 to a predetermined range, the liquid substrate adsorbed within the first distance d11 forms a liquid film to close the liquid outlet 145, and the balance is reached again, so that the liquid substrate in the first body 100 no longer continues to be added to the second liquid storage cavity 328. In this case, during use, the electronic atomization device can respond to the inhalation by the user, and automatically replenish the second body 200 with the liquid substrate from the first body 100 according to a predetermined amount in each inhalation process or during a lag period after the inhalation is completed.

In an embodiment, a volume of the first liquid storage cavity 112 in the first body 100 is greater than a volume of the second liquid storage cavity 328 in the second body 200. An amount of the liquid substrate that can be absorbed and stored by the first liquid storage cavity 112 is greater than an amount of the liquid substrate that can be absorbed and stored by the second liquid storage cavity 328 of the second body 200. For example, in some specific embodiments, the first liquid storage cavity 112 in the first body 100 can absorb and store a liquid substrate of 5-20 mL, and more specifically, for example, 10 mL. The second liquid storage cavity 328 of the second body 200 can store a liquid substrate of 5-20 mL or 0.5-3 mL, and more specifically, for example, 2 mL.

It should be noted that, the specification of this application and the accompanying drawings thereof illustrate preferred embodiments of this application, but this application is not limited to the embodiments described in the specification. Further, for a person of ordinary skill in the art, improvements or modifications may be made according to the above descriptions, and all these improvements and modifications shall fall within the protection scope of the appended claims of this application.

## Claims

1. An electronic atomization device, comprising:
a first body and a second body capable of existing independently and allowing a user operation to combine the first body with the second body, wherein:
the first body comprises:
a first liquid storage cavity, for storing a liquid substrate; and
at least one liquid output connector, configured to output the liquid substrate stored in the first liquid storage cavity,
the second body comprises:
a second liquid storage cavity, for storing a liquid substrate;
an atomization assembly, for receiving and atomizing the liquid substrate from the second liquid storage cavity to generate an aerosol; and
at least one liquid input interface, communicating with the second liquid storage cavity, and
when the first body is combined with the second body, the liquid output connector is at least partially inserted into the liquid input interface and a capillary channel is established between the liquid output connector and the liquid input interface; and the capillary channel is configured to adsorb and retain, by means of a capillarity action, the liquid substrate output from the liquid output connector, and supplement the second liquid storage cavity with the adsorbed and retained liquid substrate when an air pressure difference between the second liquid storage cavity and the first liquid storage cavity changes.

2. The electronic atomization device according to claim 1, wherein when the first body is combined with the second body, an inner surface of the liquid input interface at least partially surrounds an outer side surface of the liquid output connector, and the capillary channel is defined by a first distance between the inner surface of the liquid input interface and the outer side surface of the liquid output connector.

3. The electronic atomization device according to claim 2, wherein the first distance is in a range from 0.1 mm to 1.5 mm.

4. The electronic atomization device according to claim 2 or 3, wherein the liquid output connector has a closed free end and the outer side surface connected to the free end, and a liquid outlet for outputting a liquid substrate is provided on the outer side surface.

5. The electronic atomization device according to claim 4, wherein a diameter or a width of the liquid outlet is greater than the first distance.

6. The electronic atomization device according to claim 4, wherein a diameter or a width of the liquid outlet is in a range from 0.5 mm to 1.5 mm.

7. The electronic atomization device according to claim 4, wherein:
the liquid output connector is hollow and tubular, and a liquid output channel in communication with the first liquid storage cavity is defined in the liquid output connector; and
an inner diameter of the liquid output connector is greater than a diameter or a width of the liquid outlet.

8. The electronic atomization device according to claim 4, wherein:
at least two liquid outlets are provided on the liquid output connector; and
when one of the at least two liquid outlets outputs the liquid substrate of the first liquid storage cavity, the other liquid outlet is configured as an air inlet for air to enter the first liquid storage cavity.

9. The electronic atomization device according to claim 4, wherein:
at least two liquid outlets are provided on the liquid output connector; and
the at least two liquid outlets are arranged opposite to each other along a radial direction of the liquid output connector.

10. The electronic atomization device according to any one of claims 1 to 3, wherein the second body further comprises:
a liquid retaining element, arranged in the second liquid storage cavity, for adsorbing and retaining the liquid substrate in the second liquid storage cavity,
wherein the atomization assembly is arranged to draw or receive the liquid substrate from the liquid retaining element.

11. The electronic atomization device according to claim 10, wherein when the first body is combined with the second body, a second distance is maintained between the liquid output connector and the liquid retaining element, so that the liquid output connector and the liquid retaining element are not in contact.

12. The electronic atomization device according to claim 11, wherein the second distance is in a range from 0.1 mm to 1.5 mm.

13. The electronic atomization device according to claim 10, wherein the second body further comprises:
a first end and a second end facing away from each other along a longitudinal direction, wherein the first body is capable of being combined with the second body at the first end, wherein:
the second liquid storage cavity has a first inner wall close to the first end; and
a first compartment is provided between the liquid retaining element and the first inner wall.

14. The electronic atomization device according to claim 13, wherein:
the second liquid storage cavity further has a second inner wall close to the second end;
a second compartment is provided between the liquid retaining element and the second inner wall; and
the second compartment and the first compartment are in fluid communication.

15. The electronic atomization device according to claim 10, wherein:
the second liquid storage cavity has an inner side wall that surrounds the second liquid storage cavity along a circumferential direction;
a convex ridge extending longitudinally is provided on the inner side wall; and
an outer side surface of the liquid retaining element abuts against the convex ridge, so that an air gap is formed between the outer side surface of the liquid retaining element and the inner side wall of the second liquid storage cavity.

16. The electronic atomization device according to any one of claims 1 to 3, wherein the second body further comprises:
an airflow channel, for air to pass through the second body during inhalation; and
an air exchange channel, allowing the second liquid storage cavity and the airflow channel to be in air communication, for regulating pressure in the second liquid storage cavity.

17. The electronic atomization device according to claim 16, wherein the second body further comprises:
a support element, at least partially defining the second liquid storage cavity and the liquid input interface; and
a flexible second sealing element combined with the support element, wherein when the first body is combined with the second body, the second sealing element is at least partially located between the liquid output connector and the support element, for providing sealing between the liquid output connector and the support element.

18. The electronic atomization device according to claim 16, wherein the air exchange channel is at least partially defined between the support element and the second sealing element.

19. The electronic atomization device according to claim 16, wherein the air exchange channel comprises a vent hole or a vent slot that is formed in the support element and/or the second sealing element.

20. The electronic atomization device according to claim 16, wherein the second body further comprises:
a tubular element, running through the second liquid storage cavity along a longitudinal direction, wherein
the atomization assembly is accommodated in the tubular element and comprises:
a liquid guide element, located in the tubular element and arranged to receive the liquid substrate from the second liquid storage cavity; and
a heating element, combined with the liquid guide element and configured to heat at least part of the liquid substrate retained in the liquid guide element to generate an aerosol, wherein
the liquid guide element and/or the heating element and/or the tubular element at least partially surrounds or defines the airflow channel.

21. The electronic atomization device according to any one of claims 1 to 3, wherein the second body further comprises:
a first end and a second end facing away from each other along a longitudinal direction;
a receiving cavity that is open at the first end, wherein when the first body is combined with the second body, the first body is at least partially received in the receiving cavity; and
a cell, configured to supply power to the atomization assembly, wherein
the second liquid storage cavity is disposed between the receiving cavity and the cell.

22. The electronic atomization device according to claim 21, wherein the liquid input interface is exposed to the receiving cavity.

23. The electronic atomization device according to claim 10, wherein the liquid retaining element abuts against a port of the liquid input interface that is located in the second liquid storage cavity.

24. The electronic atomization device according to any one of claims 1 to 3, wherein:
a volume of the first liquid storage cavity is greater than a volume of the second liquid storage cavity; and/or
the first liquid storage cavity is capable of storing a liquid substrate in a range from 5mL to 20 mL; and/or
the second liquid storage cavity is capable of storing a liquid substrate in a range from 0.5 mL to 3 mL.

25. An electronic atomization device, comprising:
a first body and a second body capable of existing independently and allowing a user operation to combine the first body with the second body, wherein:
the first body comprises:
a first liquid storage cavity, for storing a liquid substrate; and
at least one liquid output connector, configured to output the liquid substrate stored in the first liquid storage cavity,
the second body comprises:
a second liquid storage cavity, for storing a liquid substrate;
an atomization assembly, for receiving and atomizing the liquid substrate from the second liquid storage cavity to generate an aerosol; and
at least one liquid input interface, communicating with the second liquid storage cavity, and
when the first body is combined with the second body, the liquid output connector is at least partially inserted into the liquid input interface to allow the first liquid storage cavity and the second liquid storage cavity to be in communication, enabling the second liquid storage cavity to be supplemented with the liquid substrate from the first liquid storage cavity; and an inner surface of the liquid input interface at least partially surrounds an outer side surface of the liquid output connector, and a first distance in a range from 0.1mm to 1.5 mm is maintained between the inner surface of the liquid input interface and the outer side surface of the liquid output connector.

26. A reservoir for an electronic atomization device, comprising:
a first body, comprising a proximal end and a distal end facing away from each other along a longitudinal direction, wherein:
a first liquid storage cavity for storing a liquid substrate is defined in the first body, and the first liquid storage cavity has an opening toward the distal end;
a closing element connected to the first body, defines at least part of a boundary of the first liquid storage cavity, and closes the opening of the first liquid storage cavity;
the closing element is provided with at least one liquid output connector extending toward the distal end; a liquid output channel is defined in the liquid output connector, and a liquid outlet is arranged on an outer side surface of the liquid output connector; and the liquid outlet is in fluid communication with the first liquid storage cavity through the liquid output channel, for outputting the liquid substrate from the first liquid storage cavity; and
a width or a diameter of the liquid outlet is less than an inner diameter of the liquid output channel.
